(19) Europäisches Patentamt
European Patent Office
Office européen des brevets

(11) **EP 2 204 512 B1**

(12) **FASCICULE DE BREVET EUROPEEN**

(45) Date de publication et mention
de la délivrance du brevet:
**25.12.2013 Bulletin 2013/52**

(51) Int Cl.:
***E04B 1/70*** *(2006.01)* ***A61N 1/16*** *(2006.01)*

(21) Numéro de dépôt: **09306348.5**

(22) Date de dépôt: **30.12.2009**

(54) **Dispositif électronique passif destiné à agir contre les remontées capillaires dans les murs et les fondations**

Passives elektronisches Gerät zum Einsatz gegen kapillares Eindringen von Feuchtigkeit in Mauern und Fundamente

Passive electronic device intended to act against capillary rise in walls and foundations

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **31.12.2008 FR 0807521**

(43) Date de publication de la demande:
**07.07.2010 Bulletin 2010/27**

(73) Titulaire: **Stumpp, Bernard**
**67300 Schiltigheim (FR)**

(72) Inventeur: **Stumpp, Bernard**
**67300 Schiltigheim (FR)**

(74) Mandataire: **Nuss, Laurent et al**
**Cabinet Nuss**
**10, rue Jacques Kablé**
**67080 Strasbourg Cedex (FR)**

(56) Documents cités:
**EP-A- 0 152 510       EP-A- 0 354 150**
**WO-A-2004/088055    AT-B- 382 915**

**Description**

La présente invention a pour objet un dispositif électronique passif destiné à agir contre les remontées capillaires dans les murs et les fondations, ce en vue de leur assèchement, ce dispositif comprenant principalement, en tant que composants fonctionnels, des circuits oscillants LC montés sur des supports isolants, lesdits circuits étant logés dans un boîtier de protection perméable aux champs électromagnétiques.

On sait que les murs des constructions, notamment des constructions anciennes, subissent souvent à leur partie inférieure, près du sol, des dégradations ou désordres dus à l'humidité qui provient généralement des remontées capillaires d'eau dans le mur.

Le phénomène de l'humidité montante par remontée capillaire dans un mur est connu sous le nom de « poussée osmotique » et varie suivant la nature des matériaux constitutifs d'un mur. Il s'explique par le fait qu'un mur est chargé positivement à sa partie inférieure humide et négativement à sa partie supérieure sèche. Ces deux zones du mur, sèche et humide, de charge électrique opposée, donnent naissance à un champ électrique qui exerce une force d'attraction sur les molécules d'eau dans les capillaires, en direction de la partie supérieure du mur.

Cependant, on a constaté que d'autres facteurs, liés à la présence de champs de stimulation électromagnétiques d'origine telluriques, interviennent également dans les remontées capillaires et que ceux-ci sont particulièrement actifs au-dessus de sources, cours d'eau souterrains, failles géologiques, zones ayant subi de forts mouvements tectoniques ou encore des zones comportant des minéraux piézo-électriques tels le quartz.

Les fréquences de ces champs électromagnétiques se situent dans tout le spectre électromagnétique allant des hautes fréquences (HF) aux micro-ondes, avec des dominantes dans les très hautes fréquences (VHF), de 30 à 300MHz, les ultra hautes fréquences (UHF), de 300 MHz à 3GHz, et les micro-ondes, notamment la bande de fréquence des micro-ondes, de 1GHz à 3GHz, qui couvre la fin de la bande de fréquence des UHF.

Ces champs sont identiques à ceux contribuant à la croissance et à la vie de la végétation en général et il est clairement établi, à ce jour, que ces champs électromagnétiques, d'origine tellurique, ont des caractéristiques différentes de celles décrites par la théorie de Maxwell.

Les travaux des professeurs ENDROS et LOTZ montrent que les cours d'eau souterrains chargés de sels minéraux et dotés de propriétés électrochimiques provoquent, en circulant à travers les sables, graviers et failles géologiques, un courant d'électricité mesurable à la surface du sol. Ce courant serait créé par la dissymétrie des charges positives et négatives des molécules d'eau, les charges négatives se fixant sur les particules du sous-sol et les charges positives, plus petites, continuant en écoulement libre.

Dans les zones construites où règnent ces activités électromagnétiques, les molécules d'eau subissent des déviations des moments magnétiques de leurs dipôles moléculaires, normalement dirigés selon le champ électromagnétique terrestre. Il se forme alors des charges électriques dans les murs traversés par ces champs électromagnétiques, dont il résulte une force électromotrice (f.é.m) et une différence de potentiel mesurable pour un mur, entre le bas de celui-ci et la limite d'humidité (frange d'humidité)

Cette force électromotrice crée à son tour un champ électrique qui donne naissance à une force mécanique, dirigée de bas en haut, d'énergie suffisante pour accroître le pompage des molécules d'eau vers le haut du mur, jusqu'à leur niveau d'équilibre.

C'est pour cette raison que l'on observe souvent que les remontées d'eau par capillarité sont irrégulières sur les murs (hauteur de la frange d'humidité) bien que la nature des sols et des matériaux en présence soit identique.

Actuellement, pour combattre l'humidité montante, les procédés nécessitent des interventions manuelles et des travaux lourds et coûteux sur les murs ou, dans certains cas, l'utilisation de produits chimiques néfastes pour l'environnement.

On connaît également des dispositifs dits « électro-physique » tels que celui décrit dans le document EP-A-0 152 510 qui comporte des selfs et des condensateurs électriques montés sur des supports isolants et reliés parallèlement entre eux, l'ensemble étant logé dans un coffret perméable aux champs électromagnétiques.

Mais un tel appareil agit seulement sur les champs électromagnétiques dont les fréquences sont situées dans une bande de fréquence comprise entre 10 kHz et 150 MHz, excluant, d'une part, les fréquences VHF de 150 MHz à 300 MHz, lesquelles sont également en cause dans les remontées capillaires comme l'a constaté le présent inventeur, et, d'autre part, les fréquences UHF et les micro-ondes, au-delà de 300 MHz.

Le document EP 0 354 150 décrit un dispositif capable de neutraliser efficacement les effets des champs de stimulation électromagnétiques telluriques exerçant le pompage de l'eau vers le haut du mur, dans les rayonnements électromagnétiques situés dans les fréquences basses, VHF, UHF et les micro-ondes. Il comporte des circuits oscillants parallèles, à savoir au moins quatre selfs reliées respectivement à au moins quatre condensateurs électriques, montés sur des supports isolants et reliés parallèlement entre eux, et disposés en deux paires de selfs comportant, chacune, une self interne et une self externe, chaque self interne étant reliée, électriquement, d'une part, par ses deux extrémités au condensateur correspondant et, d'autre part, par une extrémité à la self externe correspondante, ces selfs externes étant, en outre, reliées chacune par leurs deux extrémités à un dipôle rayonnant correspondant, qui est lui-même relié électriquement, par ses deux pôles, au condensateur correspondant, chaque self externe étant ainsi, également, reliée, électriquement, à chaque condensateur. L'ensemble des quatre circuits oscillants parallèles ainsi formés et associés aux dipôles rayonnants est logé dans un coffret de protection perméable aux champs électromagnétiques et a pour effet de créer un contrechamp déphasé électriquement par rapport aux champs initiaux de stimulation électromagnétique, de façon à annuler l'effet de pompage de l'eau vers le haut des murs.

Toutefois, ce dispositif agit uniquement, dans la bande de fréquence des VHF, jusqu'à environ 150 MHz et, seulement, dans les bandes de fréquence des UHF et des micro-ondes, jusqu'à environ 2 GHz.

On connaît également des dispositifs électroniques actifs alimentés en énergie et permettant la création de champs électromagnétiques.

Cependant, ces dispositifs électroniques actifs qui sont consommateurs d'énergie n'offrent pas une grande autonomie et présentent des constitutions plus complexes.

La présente invention a pour but de remédier à ces inconvénients en proposant un dispositif contre les remontées capillaires capable de couvrir un spectre de fréquence, dans les bandes de fréquence VHF, UHF et des micro-ondes, plus large, en valeur supérieure, que les dispositifs actuels, à savoir, notamment, au-delà de 150 MHz pour la bande de fréquence VHF et au-delà de 2 GHz pour la bande de fréquence des micro-ondes.

Le dispositif électronique passif selon l'invention destiné à agir contre les remontées capillaires dans les murs et les fondations, ce en vue de leur assèchement, est du type comprenant principalement, en tant que composants fonctionnels, des circuits oscillants LC montés sur des supports isolants, lesdits circuits étant logés dans un boîtier de protection perméable aux champs électromagnétiques, et se caractérise, comme l'indique la revendication 1, en ce qu'il comprend au moins un circuit oscillant primaire apte à neutraliser les rayonnements électromagnétiques d'origine tellurique dans la bande de fréquence VHF, au moins un circuit oscillant primaire apte à neutraliser les rayonnements électromagnétiques d'origine tellurique dans la bande de fréquence UHF et au moins un circuit oscillant primaire apte à neutraliser les rayonnements électromagnétiques d'origine tellurique dans le domaine des micro-ondes, lesdits circuits oscillants primaires étant, matériellement ou structurellement, indépendants les uns des autres et constitués, chacun, d'une self primaire reliée électriquement à un condensateur primaire et/ou associée à la capacité parasite du circuit considéré et en ce que lesdites selfs primaires, à structure sensiblement plate, sont situées approximativement dans un même plan de référence.

Le dispositif selon l'invention permet notamment de corriger et de réorienter naturellement les molécules d'eau pour qu'elles ne soient plus poussées vers le haut d'un mur chargé négativement.

Des caractéristiques additionnelles et des variantes de réalisation du dispositif selon l'invention sont évoquées dans les revendications dépendantes.

L'invention sera mieux comprise, grâce à la description ci-après, qui se rapporte à plusieurs modes de réalisation, donnés à titre d'exemples non limitatifs, et expliqués avec références aux dessins schématiques annexés, dans lesquels :

- la figure 1 est une vue de dessus d'un premier mode de réalisation du dispositif selon l'invention, hors coffret de protection,
- les figures 2a, 2b et 2c sont des vues de profil, respectivement, de trois circuits oscillants primaires, du dispositif de la figure 1, associés, chacun, à un circuit oscillant complémentaire,
- les figures 3a, 3b et 3c sont des vues de profil, respectivement, de trois circuits oscillants primaires, dans un deuxième mode de réalisation de l'invention, associés, chacun, à un circuit oscillant complémentaire,
- la figure 4a est une vue de profil schématique de la position relative d'un circuit oscillant primaire par rapport au circuit oscillant complémentaire associé, selon l'invention, dans une disposition coaxiale,
- la figure 4b est une vue de profil schématique de la position relative d'un circuit oscillant primaire par rapport au circuit oscillant complémentaire associé, selon l'invention, dans une disposition décalée axialement,
- la figure 5 est une vue de profil de deux supports isolants plans inclinés par rapport au plan de référence,
- la figure 6 est une vue en plan (sans le support isolant), dans un mode de réalisation alternatif, de la structure spiralée plane d'une self primaire respectivement complémentaire pouvant faire partie d'un circuit oscillant primaire apte à neutraliser les rayonnements électromagnétiques d'origine tellurique dans le domaine des micro-ondes.

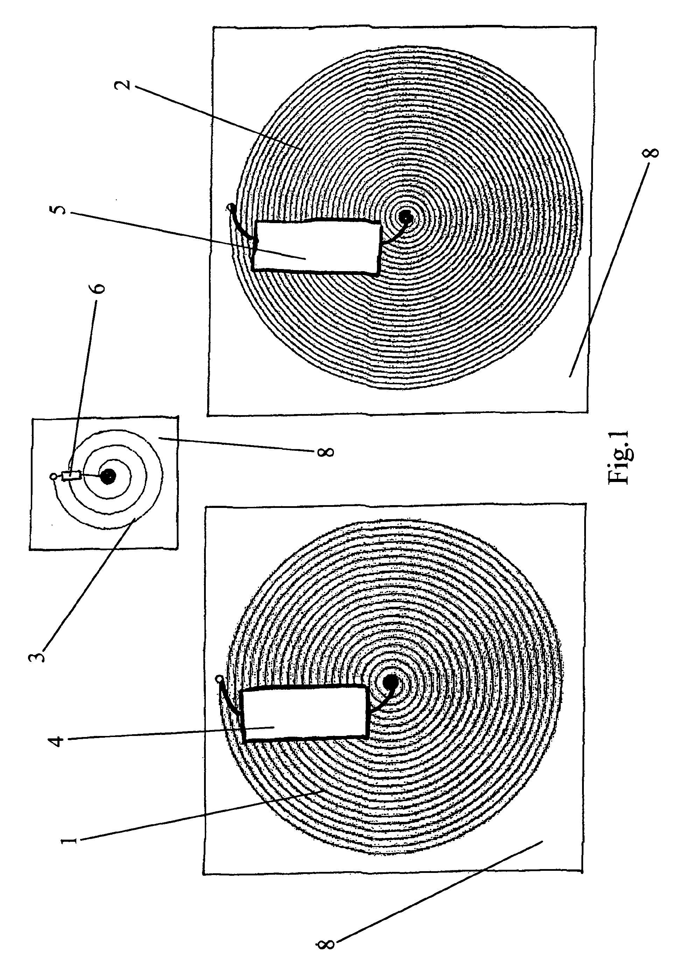

En se référant à la figure 1, on peut voir que le dispositif électronique passif destiné à agir contre les remontées capillaires dans les murs et les fondations, ce en vue de leur assèchement, comprend principalement, en tant que composants fonctionnels, des circuits oscillants LC 1, 4; 2, 5; 3, 6 montés sur des supports isolants, lesdits circuits étant logés dans un boîtier, non représenté, perméable aux champs électromagnétiques donc ne présentant pas de blindage électrique.

Le dispositif selon l'invention est **caractérisé en ce qu'**il comprend au moins un circuit oscillant primaire 2, 5 apte à neutraliser les rayonnements électromagnétiques d'origine tellurique dans la bande de fréquence VHF, au moins un circuit oscillant primaire 1, 4 apte à neutraliser les rayonnements électromagnétiques d'origine tellurique dans la bande de fréquence UHF et au moins un circuit oscillant primaire 3, 6 ou 10 apte à neutraliser les rayonnements électromagnétiques d'origine tellurique dans le domaine des micro-ondes.

On peut voir également, sur les différentes figures, que, d'une part, les circuits oscillants primaires 1, 4; 2, 5; 3, 6 ou 10, montés chacun sur un support isolant 8, sont indépendants les uns des autres (structurellement et au niveau de leur positionnement notamment) et constitués, chacun, d'une self primaire 1, 2, 3 reliée électriquement à un condensateur primaire 4, 5, 6 et/ou associée à la capacité parasite du circuit considéré et, d'autre part, les selfs primaires 1, 2, 3 ou 10, à structure sensiblement plate, sont situées, approximativement, dans un même plan de référence.

On entendra par circuits oscillants primaires 1, 4; 2, 5; 3, 6 ou 10 indépendants les uns des autres, des circuits qui n'ont pas de liens entre eux électriquement et, partant, des circuits qui n'ont pas, ou sensiblement pas, d'interactions, notamment électromagnétiques, les uns avec les autres.

De même, en étant montés sur des supports séparés, les circuits oscillants primaires peuvent être positionnés indépendamment les uns des autres, même en étant éventuellement logés dans le même boîtier.

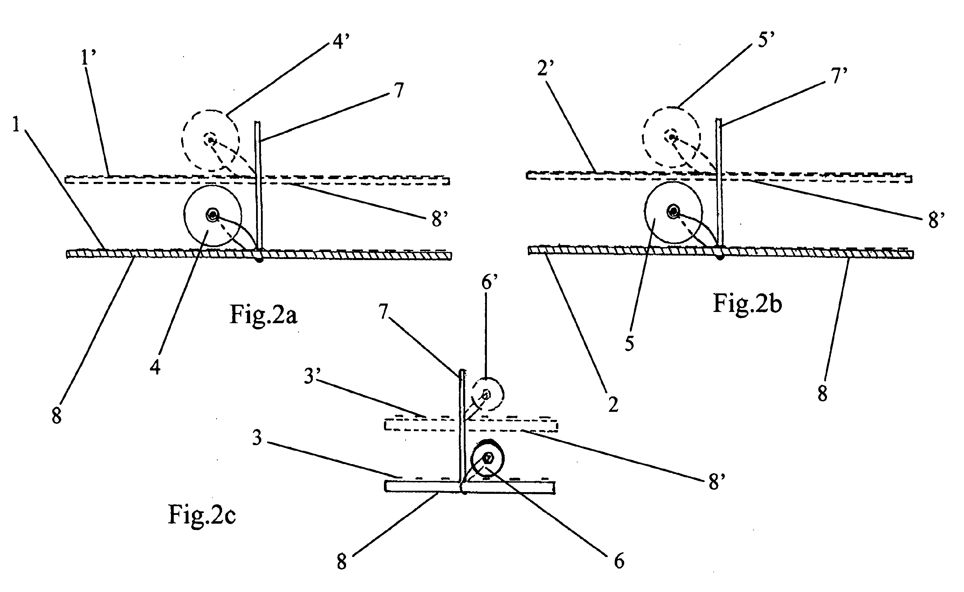

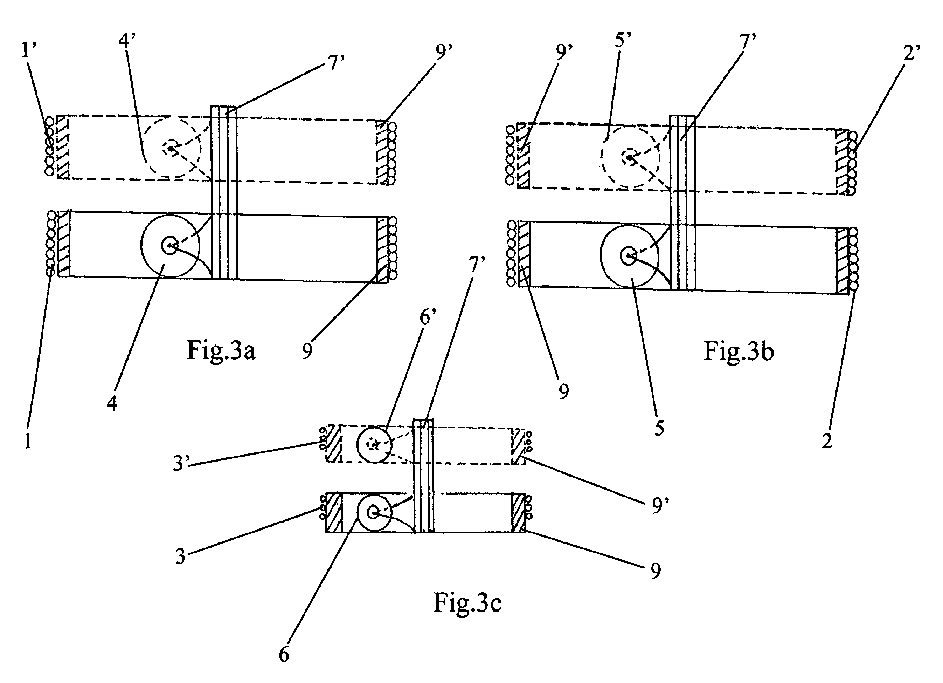

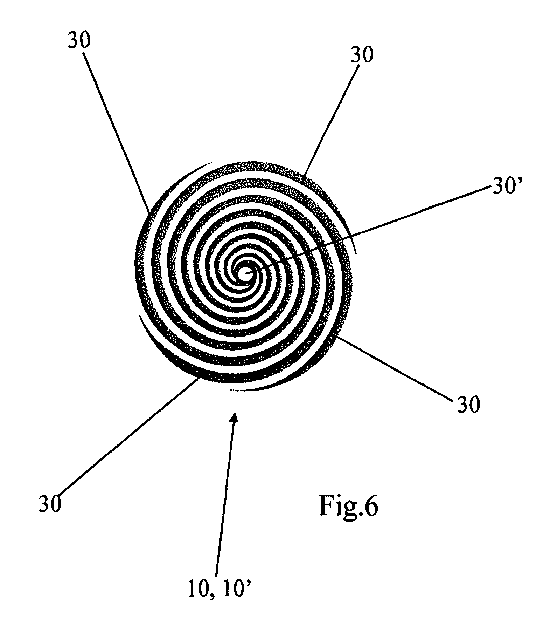

Si on se réfère maintenant aux figures 2a, 2b et 2c et aux figures 3a, 3b et 3c, et par extension à la figure 6 (en tant qu'alternative partielle), on peut voir qu'à chaque circuit oscillant primaire 1, 4; 2, 5; 3, 6 peut être associé, respectivement, au moins un circuit oscillant complémentaire 1', 4'; 2', 5'; 3', 6' ou 10', monté sur un support isolant 8'.

Chaque circuit oscillant complémentaire 1', 4'; 2', 5'; 3', 6' ou 10' comporte une self complémentaire 1', 2', 3' ou 10' reliée, électriquement, à un condensateur complémentaire 4', 5', 6' et/ou associée à la capacité parasite du circuit considéré et chaque self complémentaire 1', 2', 3' ou 10' peut être disposée sensiblement parallèlement à la self primaire 1, 2, 3 ou 10 associée du circuit oscillant primaire 1, 4; 2, 5; 3, 6 ou 10 correspondant.

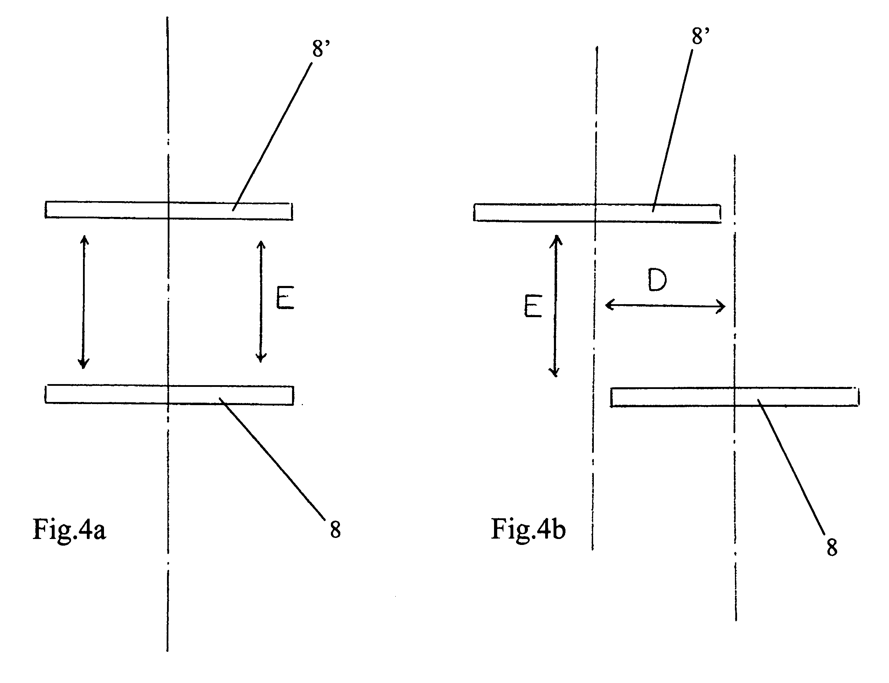

D'autre part, chaque self complémentaire 1', 2', 3' ou 10' d'un circuit oscillant complémentaire 1', 4'; 2', 5'; 3', 6' ou 10', représenté schématiquement sur la figure 4a et sur la figure 4b par son support isolant 8' et sur la figure 6, peut présenter une structure et un positionnement par rapport à la self primaire 1, 2, 3 ou 10, du circuit oscillant primaire 1, 4; 2, 5; 3, 6 ou 10 correspondant, qui sont fonction du diagramme de rayonnement recherché et des fréquences à neutraliser. Ainsi, une telle self complémentaire 1', 2', 3' ou 10', peut être, par rapport à la self primaire 1, 2, 3 ou 10, du circuit oscillant 1, 4; 2, 5; 3, 6 ou 10 primaire correspondant, identique ou différente, disposée co-axialement (figure 4a) ou décalée axialement (D) dans l'espace (figure 4b), en contact direct ou avec un écartement E, de préférence, inférieur à environ 170 mm.

Les circuits oscillants complémentaires 1', 4'; 2', 5'; 3', 6' ou 10' ont pour fonction d'augmenter l'efficacité du dispositif selon la présente invention et, plus particulièrement, d'augmenter le rendement et l'intensité du contre champ électromagnétique et de modifier le diagramme de rayonnement.

Le calcul de la fréquence d'oscillation des circuits oscillants primaires 1, 4; 2, 5; 3, 6 ou 10, respectivement complémentaire 1', 4'; 2', 5'; 3', 6' ou 10', dans les différentes bandes de fréquence se fait selon la formule utilisée en radio-technique (confer document EP 0 354 150), à savoir :

$$F = 1/2\pi\sqrt{LC}$$

où:

    F : Fréquence
    L : Inductance
    C : Capacité

Le nombre de spires de chaque self primaire 1, 2, respectivement complémentaire 1', 2', d'un circuit oscillant primaire 1, 4 ; 2, 5, respectivement complémentaire 1', 4'; 2', 5' apte à neutraliser les rayonnements électromagnétiques d'origine tellurique dans la bande de fréquence VHF ou UHF correspondante, est compris entre 8 et 60 spires, préférentiellement entre 10 et 48 spires, et la valeur de la capacité d'un condensateur primaire 4, 5, respectivement complémentaire 4', 5', relié, électriquement, à ladite self primaire 1, 2, respectivement complémentaire 1', 2', est avantageusement comprise entre 0,01 et 3$\mu$F, préférentiellement entre 0.1 et 2$\mu$F. Par ailleurs, le nombre de spires de la self primaire 3, respectivement complémentaire 3', du circuit oscillant primaire 3, 6, respectivement complémentaire 3', 6', apte à neutraliser les rayonnements dans le domaine des micro-ondes, est avantageusement compris entre 1 et 5 spires, préférentiellement entre 1 et 3 spires, et la valeur de la capacité du condensateur primaire 6, respectivement complémentaire 6' relié, électriquement, à ladite self primaire 3, respectivement complémentaire 3', est avantageusement comprise entre 1 et 22 pF. La capacité du condensateur 6, respectivement 6' peut être constituée uniquement par la capacité parasite, qui est alors associée à la self 3, 3' pour former le circuit oscillant considéré.

Par ailleurs, le diamètre de chaque self primaire 1, 2, 3, respectivement, complémentaire 1', 2', 3' est, avantageusement, compris entre 50 et 260 mm et le pas de chaque self primaire 1, 2, 3, respectivement, complémentaire 1', 2', 3' est, avantageusement, compris entre 0,2 et 6 mm.

Chaque circuit oscillant primaire 1, 4; 2, 5; 3, 6 ou 10, respectivement complémentaire 1', 4'; 2', 5'; 3', 6' ou 10', peut être monté sur un support isolant plan primaire 8, respectivement complémentaire 8', en sorte que chaque self primaire 1, 2, 3 ou 10, respectivement complémentaire 1', 2', 3' ou 10', présente une structure spiralée plane (figure 2a, figure 2b, figure 2c et figure 6).

La structure spiralée plane de chaque self primaire 1, 2, 3 ou 10, respectivement complémentaire 1', 2', 3' ou 10', peut être réalisée à partir d'au moins un conducteur de cuivre ou d'au moins une bande de cuivre étamée, argentée ou dorée, disposé(e) à même le support isolant plan primaire 8, respectivement complémentaire 8' correspondant.

Chaque condensateur primaire 4, 5, 6, respectivement complémentaire 4', 5', 6', peut être relié, électriquement, au centre et à l'extrémité de la self primaire 1, 2, 3, respectivement complémentaire 1', 2', 3', correspondante du circuit oscillant concerné.

Comme le montre schématiquement et partiellement la figure 6 des dessins, et en remplacement des circuits primaires 3, 6 et le cas échéant complémentaires 3', 6', il peut être prévu de manière alternative que la structure spiralée plane de la self primaire 10 respectivement complémentaire 10' du au moins un circuit oscillant primaire 10 respectivement complémentaire 10' apte à neutraliser les rayonnements électromagnétiques d'origine tellurique dans le domaine des micro-ondes comprend plusieurs bras spiralés 30 imbriqués les uns dans les autres de manière alternée et reliés électriquement entre eux à leur extrémité intérieure formant le point central de révolution ou centre 30' desdits bras spiralés 3 et que, d'une part, chaque bras spiralé 30 s'étend sur 1 à 3 tours et, d'autre part, le pas de la structure spiralée ainsi obtenue est constant ou augmente progressivement depuis ledit point central 30'.

Les bras spiralés 30 sont avantageusement au nombre de 2 à 5.

En accord avec la variante de réalisation précitée, les selfs primaire 10 et complémentaire 10' sont identiques et forment chacune respectivement un circuit oscillant primaire ou un circuit oscillant complémentaire, ce en association avec une capacité parasite correspondante présente au niveau dudit circuit. Dans le cadre de cette variante, les circuits oscillants 1, 4 et 2, 5 neutralisant les rayonnements VHF et UHF peuvent présenter la structure et la constitution définies précédemment.

Le centre d'une self primaire 1, 2, 3 ou 10, respectivement complémentaire 1', 2', 3' ou 10', peut être relié, en outre à, un brin métallique conducteur, un barreau de ferrite 7, un barreau aimanté ou un quartz, de quelques millimètres à quelques centimètres de longueur.

Le brin métallique conducteur, le barreau de ferrite 7 respectivement 7', le barreau aimanté ou le quartz, peut être positionné, perpendiculairement, au support isolant plan primaire 8, respectivement complémentaire 8', correspondant, ou intégré, perpendiculairement, dans ledit support isolant plan primaire 8, respectivement complémentaire 8', en sorte que la longueur émergeant au-dessus du support isolant plan primaire 8, respectivement complémentaire 8', soit comprise entre environ 40 et 100 % de la longueur totale.

Dans une variante, chaque self primaire 1, 2, 3, respectivement complémentaire 1', 2', 3', peut présenter une structure spiralée cylindrique (figure 3a, figure 3b et figure 3c) et les spires de chaque self primaire 1, 2, 3, respectivement complémentaire 1', 2', 3', sont alors bobinées sur un support isolant cylindrique primaire 9, respectivement complémentaire 9'.

Les selfs 1, 1', 2, 2', 3 et 3' constituent dans ce cas, avec les supports 9 et 9', des structures cylindriques plates, c'est-à-dire de faible hauteur ou épaisseur axiale par rapport à leur diamètre.

On notera que, conformément à l'invention, les supports isolants représentés sur la figure 4a et sur la figure 4b peuvent être, selon la structure desdits supports, des supports isolants plans 8, 8', tels que représentés schématiquement, ou des supports isolants cylindriques plats 9, 9'.

Dans une autre forme de réalisation, chaque self primaire 1, 2, 3, respectivement complémentaire 1', 2', 3', peut présenter une structure spiralée cylindrique plate (c'est-à-dire de faible hauteur) et les spires de chaque self primaire 1, 2, 3, respectivement complémentaire 1', 2', 3', peuvent être constituées par un fil rigide et autoportant.

Chaque condensateur primaire 4, 5, 6, respectivement complémentaire 4', 5', 6', est avantageusement relié, électriquement, à l'extrémité basse et à l'extrémité haute de la self primaire 1, 2, 3, respectivement complémentaire 1', 2', 3', correspondante du circuit oscillant concerné.

Un barreau de ferrite, un barreau aimanté ou un quartz, de quelques millimètres à quelques centimètres de longueur, peut être positionné, sensiblement axialement, à l'intérieur de la structure cylindrique plate de chaque self primaire 1, 2, 3, respectivement complémentaire 1', 2', 3'. Un brin métallique conducteur peut être fixé à l'extrémité haute de chaque self primaire 1, 2, 3, respectivement complémentaire 1', 2', 3', correspondante du circuit oscillant concerné.

Conformément à l'invention un brin, un barreau de ferrite ou un barreau aimanté est, avantageusement, fixé à un support isolant correspondant par l'intermédiaire d'une bague de serrage, laquelle bague étant, de préférence, une bague métallique pouvant être soudée audit support isolant correspondant.

Toujours conformément à l'invention, un brin, un barreau de ferrite et un barreau aimanté auront chacun pour fonction d'augmenter l'inductance du circuit oscillant primaire 1, 4; 2, 5; 3, 6 ou 10, respectivement complémentaire 1', 4'; 2', 5'; 3', 6' ou 10' correspondant, tandis qu'un quartz aura pour fonction d'homogénéiser le contre champ électromagnétique.

Dans une première configuration, deux supports isolants, qui peuvent être des supports isolants plans primaires 8 ou des supports isolants cylindriques primaires 9, respectivement complémentaire 8' ou 9', des circuits oscillants primaires 1, 2; 4, 5, respectivement complémentaires 1', 2'; 4', 5', aptes à neutraliser les rayonnements électromagnétiques telluriques dans les bandes de fréquence VHF et UHF, sont inclinés, chacun, d'un angle proche de ou sensiblement égal à 0° par rapport au plan de référence P.

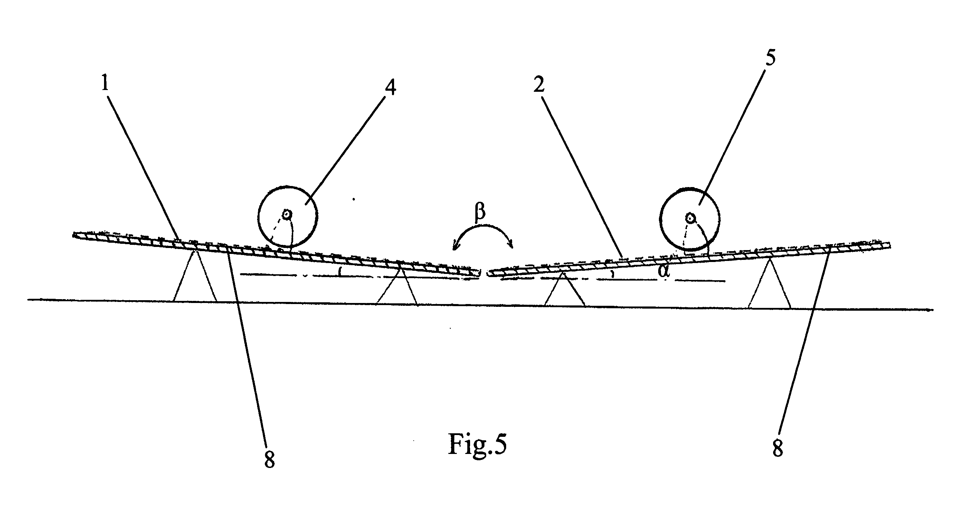

Dans une deuxième configuration, comme on peut le voir sur la figure 5, deux supports isolants, qui peuvent être des supports isolants plans primaires 8 ou des supports isolants cylindriques primaires 9, respectivement complémentaire 8' ou 9', des circuits oscillants primaires 1, 2, 4, 5, respectivement complémentaires 1', 2'; 4', 5', aptes à neutraliser les rayonnements électromagnétiques telluriques dans les bandes de fréquence VHF et UHF, sont inclinés, chacun, d'un angle $\alpha$, approximativement de 1 ° à 30°, préférentiellement de 1° à 20°, par rapport au plan de référence P, ceci de manière à former une configuration en V entre lesdits supports isolants 8 ou 9, respectivement complémentaires 8' ou 9', d'un angle $\beta$, approximativement, de 120° à 180°. Cette configuration permet ainsi une augmentation de l'angle d'émission et de réception $\theta$, complémentaire de l'angle $\beta$ sur 360°. L'ouverture en V est orientée, soit vers le bas pour concentrer les champs électromagnétiques, soit vers le haut pour disperser lesdits champs électromagnétiques.

Dans les deux configurations décrites précédemment, un support isolant, qui peut être un support isolant plan primaire 8 ou un support isolant cylindrique primaire 9, respectivement complémentaire 8' ou 9', d'un circuit oscillant primaire 3, 6 ou 10, respectivement complémentaire 3', 6' ou 10', apte à neutraliser les rayonnements électromagnétiques telluriques dans les micro-ondes est, de préférence, situé dans ledit plan de référence P.

La capacité C de chaque circuit oscillant LC primaire 3, 6 ou 10, respectivement complémentaire 3', 6' ou 10', apte à neutraliser les rayonnements électromagnétiques d'origine tellurique dans les micro-ondes, peut être uniquement constitué par la capacité parasite dudit circuit oscillant LC primaire 3, 6; 10, respectivement complémentaire 3', 6'; 10. La capacité parasite est entendue comme étant la ou les capacités parasites du circuit concerné.

Ainsi un tel circuit LC oscillant possède une pulsation $\omega$ :

$$\omega = 1/\sqrt{LC} \, ;$$

soit une fréquence F:

$$F = \omega / 2\pi = 1/2\pi\sqrt{LC} \, ,$$

où C est la valeur équivalente de la capacité parasite.

Conformément à l'invention, chaque circuit oscillant complémentaire 1', 2'; 3', 4'; 5', 6' ou 10' peut être relié, électriquement, au circuit oscillant primaire 1, 2; 3, 4; 5, 6 ou 10 auquel il est associé, par l'intermédiaire de l'une des extrémités de la self complémentaire 1 ; 2'; 3' ou 10', à l'une des extrémités, intérieure ou extérieure, de la self primaire 1; 2; 3 ou 10.

D'autre part, chaque circuit oscillant primaire 1, 2; 3, 4; 5, 6 ou 10, respectivement complémentaire 1', 2'; 3', 4', 5', 6' ou 10', peut être coulé dans une résine isolante, telle qu'une résine époxy ou polyuréthane, de manière à les protéger contre l'oxydation et l'action de l'humidité.

Enfin, selon une caractéristique additionnelle de l'invention, il peut être prévu que les circuits oscillants primaires 1, 4, et 2, 5, et le cas échéant complémentaires 1', 4' et 2', 5', aptes à neutraliser les rayonnements électromagnétiques dans les bandes de fréquences VHF et UHF comportent en outre des selfs extérieures, comprenant une ou deux spires chacune, arrangées respectivement autour des selfs primaires 1 ou 2, et le cas échéant complémentaires 1' ou 2', desdits circuits oscillants et munies à leurs extrémités de brins rayonnants ou analogues, tels que décrits précédemment.

En ce qui concerne l'installation du dispositif selon la présente invention, celui-ci est plus particulièrement destiné à être soit disposé contre un mur soit noyé dans un mur lorsque ce dernier est épais.

En fonction du diagramme de rayonnement recherché et des fréquences spécifiques à neutraliser, qui sont propres à chaque site d'installation du dispositif selon l'invention, l'opérateur spécialisé dans le domaine considéré adapte la structure de chaque self complémentaire et son positionnement par rapport à la self principale correspondante. Cet ajustement sera d'autant plus rapide et aisé que l'opérateur sera expérimenté et bien formé.

**Revendications**

1. Dispositif électronique passif destiné à agir contre les remontées capillaires dans les murs et les fondations, ce en vue de leur assèchement, ce dispositif comprenant principalement, en tant que composants fonctionnels, des circuits oscillants LC, lesdits circuits oscillants (1, 4; 2, 5; 3, 6 ou 10) étant indépendants les uns des autres, logés dans un boîtier de protection perméable aux champs électromagnétiques et constitués, chacun, d'une self primaire (1; 2; 3 ou 10) reliée électriquement à un condensateur primaire (4, 5, 6) et/ou associée à la capacité parasite du circuit considéré et en ce que lesdites selfs primaires (1; 2; 3 ou 10), à structure sensiblement plate, sont situées approximativement dans un même plan de référence (P),

   dispositif **caractérisé**

   **en ce que** lesdits circuits oscillants (1, 4; 2, 5; 3, 6 ou 10) sont montés sur des supports isolants,

   **en ce qu'**il comprend au moins un circuit oscillant primaire (2, 5) apte à neutraliser les rayonnements électromagnétiques d'origine tellurique dans la bande de fréquence VHF, au moins un circuit oscillant primaire (1, 4) apte à neutraliser les rayonnements électromagnétiques d'origine tellurique dans la bande de fréquence UHF et au moins un circuit oscillant primaire (3, 6; 10) apte à neutraliser les rayonnements électromagnétiques d'origine tellurique dans le domaine des micro-ondes,

**en ce qu'**à chaque circuit oscillant primaire (1, 4; 2, 5; 3, 6 ou 10) est associé, respectivement, au moins un circuit oscillant complémentaire (1', 4'; 2', 5'; 3', 6' ou 10'), comportant chacun une self complémentaire (1', 2', 3' ou 10') reliée, électriquement, à un condensateur complémentaire (4', 5', 6') et/ou associée à la capacité parasite du circuit considéré, et **en ce que** chaque self complémentaire (1' ; 2' ; 3' ou 10') est disposée sensiblement parallèlement à la self primaire (1 ; 2 ; 3 ou 10) associée du circuit oscillant primaire (1, 4; 2, 5; 3, 6 ou 10) correspondant et présente une structure et un positionnement par rapport à la self primaire (1 ; 2 ; 3 ou 10) correspondante qui sont fonction du diagramme de rayonnement recherché et des fréquences à neutraliser.

2. Dispositif, selon la revendication 1, **caractérisé en ce que** le nombre de spires de chaque self primaire (1, 2), respectivement complémentaire (1', 2'), d'un circuit oscillant primaire (1, 4; 2, 5), respectivement complémentaire (1', 4'; 2', 5') apte à neutraliser les rayonnements électromagnétiques d'origine tellurique dans la bande de fréquence VHF ou UHF correspondante, est compris entre 8 et 60 spires, préférentiellement entre 10 et 48 spires, et la valeur de la capacité d'un condensateur primaire (4, 5), respectivement complémentaire (4', 5'), relié, électriquement, à ladite self primaire (1, 2), respectivement complémentaire (1', 2'), est avantageusement comprise entre 0,01 et 3$\mu$F, préférentiellement entre 0.1 et 2$\mu$F, et **en ce que** le nombre de spires de la self primaire (3), respectivement complémentaire (3'), du circuit oscillant primaire (3, 6), respectivement complémentaire (3', 6'), apte à neutraliser les rayonnements dans le domaine des micro-ondes, est compris entre 1 et 5 spires, préférentiellement entre 1 et 3 spires, et la valeur de la capacité du condensateur primaire (6), respectivement complémentaire (6') relié électriquement à ladite self primaire (3), respectivement complémentaire (3'), ou associé à cette self (3, 3') sous forme de capacité parasite est comprise entre 1 et 22 pF.

3. Dispositif, selon la revendication 2, **caractérisé en ce que** le diamètre de chaque self primaire (1, 2, 3), respectivement, complémentaire (1', 2', 3') est compris entre 50 et 260 mm et **en ce que** le pas de chaque self primaire (1, 2, 3), respectivement, complémentaire (1', 2', 3') est compris entre 0,2 et 6 mm.

4. Dispositif, selon l'une quelconque des revendication 1 à 3, **caractérisé en ce que** chaque circuit oscillant primaire (1, 4; 2, 5; 3, 6 ou 10), respectivement complémentaire (1', 4'; 2', 5'; 3', 6' ou 10'), est monté sur un support isolant plan primaire (8), respectivement complémentaire (8'), en sorte que chaque self primaire (1 ; 2 ; 3 ou 10), respectivement complémentaire (1' ; 2' ; 3' ou 10'), présente une structure spiralée plane.

5. Dispositif, selon la revendication 4, **caractérisé en ce que** la structure spiralée plane de chaque self primaire (1 ; 2 ; 3 ou 10), respectivement complémentaire (1' ; 2' ; 3' ou 10'), est réalisée à partir d'au moins un conducteur de cuivre ou d'au moins une bande de cuivre étamée, argentée ou dorée, disposé(e) à même le support isolant plan primaire (8), respectivement complémentaire (8') correspondant, et enroulé(e) en spirale.

6. Dispositif, selon la revendication 4 ou la revendication 5, **caractérisé en ce que** chaque condensateur primaire (4, 5, 6), respectivement complémentaire (4', 5', 6'), est relié, électriquement, au centre et à l'extrémité libre de la self primaire (1, 2, 3), respectivement complémentaire (1', 2', 3'), correspondante du circuit oscillant concerné.

7. Dispositif selon la revendication 4 ou la revendication 5, **caractérisé en ce que** la structure spiralée plane de la self primaire (10) respectivement complémentaire (10') du au moins un circuit oscillant primaire (10) respectivement complémentaire (10') apte à neutraliser les rayonnements électromagnétiques d'origine tellurique dans le domaine des micro-ondes comprend plusieurs bras spiralés (30) imbriqués les uns dans les autres de manière alternée et reliés électriquement entre eux à leur extrémité intérieure formant le point central de révolution ou centre (30') desdits bras spiralés (3) et **en ce que**, d'une part, chaque bras spiralé (30) s'étend sur 1 à 3 tours et, d'autre part, le pas de la structure spiralée ainsi obtenue est constant ou augmente progressivement depuis ledit point central (30').

8. Dispositif, selon la revendication 7, **caractérisé en ce que** les selfs primaire (10) et complémentaire (10') sont identiques et forment chacune respectivement un circuit oscillant primaire ou un circuit oscillant complémentaire, ce en association avec une capacité parasite correspondante présente au niveau dudit circuit.

9. Dispositif, selon la revendication 6 ou la revendication 8, **caractérisé en ce que** le centre d'une self primaire (1, 2, 3, 10), respectivement complémentaire (1', 2', 3', 10'), est relié, en outre à, un brin métallique conducteur, un barreau de ferrite (7), un barreau aimanté ou un quartz, de quelques millimètres à quelques centimètres de longueur.

10. Dispositif, selon la revendication 9, **caractérisé en ce que** le brin métallique conducteur, le barreau de ferrite (7), le barreau aimanté ou le quartz, est positionné, perpendiculairement, au support isolant plan primaire (8), respectivement complémentaire (8'), correspondant, ou intégré, perpendiculairement, dans ledit support isolant plan primaire (8), respectivement complémentaire (8'), en sorte que la longueur émergeant au-dessus du support isolant plan primaire (8), respectivement complémentaire (8'), soit comprise entre environ 40 et 100 % de la longueur totale.

11. Dispositif, selon l'une quelconque des revendications 1 à 3, **caractérisé en ce que** chaque self primaire (1, 2, 3), respectivement complémentaire (1', 2', 3'), présente une structure spiralée cylindrique et **en ce que** les spires de chaque self primaire (1, 2, 3), respectivement complémentaire (1', 2', 3'), sont bobinées sur un support isolant cylindrique primaire (9), respectivement complémentaire (9').

12. Dispositif, selon l'une quelconque des revendications 1 à 3, **caractérisé en ce que** chaque self primaire (1, 2, 3), respectivement complémentaire (1', 2', 3'), présente une structure spiralée cylindrique plate et **en ce que** les spires de chaque self primaire (1, 2, 3), respectivement complémentaire (1', 2', 3'), sont constituées par un fil rigide et autoportant

13. Dispositif, selon la revendication 11 ou la revendication 12, **caractérisé en ce que** chaque condensateur primaire (4, 5, 6), respectivement complémentaire (4', 5', 6'), est relié, électriquement, à l'extrémité basse et à l'extrémité haute de la self primaire (1, 2, 3), respectivement complémentaire (1', 2', 3'), correspondante du circuit oscillant concerné.

14. Dispositif, selon l'une quelconque des revendications 11 à 13, **caractérisé en ce qu'**un barreau de ferrite, un barreau aimanté ou un quartz, de quelques millimètres à quelques centimètres de longueur, est positionné, sensiblement axialement, à l'intérieur de la structure cylindrique plate de chaque self primaire (1, 2, 3), respectivement complémentaire (1', 2', 3').

15. Dispositif, selon l'une quelconque des revendications 11 à 14, **caractérisé en ce qu'**un brin métallique conducteur est fixé à l'extrémité haute de chaque self primaire (1, 2, 3), respectivement complémentaire (1', 2', 3'), correspondante du circuit oscillant concerné.

16. Dispositif, selon l'une quelconque des revendications 1 à 15, **caractérisé en ce que** les supports isolants primaires (8; 9), respectivement complémentaires (8'; 9'), des circuits oscillants primaires (1, 2; 4, 5), respectivement complémentaires (1', 2'; 4', 5'), aptes à neutraliser les rayonnements électromagnétiques telluriques dans les bandes de fréquence VHF et UHF, sont inclinés d'un angle proche de ou sensiblement égal à 0° par rapport au plan de référence (P) et **en ce qu'**un support isolant primaire (8; 9), respectivement complémentaire (8'; 9'), d'un circuit oscillant primaire (3, 6 ou 10), respectivement complémentaire (3', 6' ou 10'), apte à neutraliser les rayonnements électromagnétiques telluriques dans les micro-ondes est, de préférence, situé dans ledit plan de référence (P).

17. Dispositif, selon l'une quelconque des revendications 1 à 15, **caractérisé en ce que** les supports isolants primaires (8; 9), respectivement complémentaires (8'; 9'), des circuits oscillants primaires (1, 2; 4, 5), respectivement complémentaires (1', 2'; 4', 5'), aptes à neutraliser les rayonnements électromagnétiques telluriques dans les bandes de fréquence VHF et UHF, sont inclinés d'un angle ($\alpha$), approximativement de 1° à 30°, préférentiellement de 1° à 20°, par rapport au plan de référence (P), ceci de manière à former une configuration en V entre lesdits supports isolants primaires (8; 9), respectivement complémentaires (8'; 9'), d'un angle ($\beta$), approximativement de 120° à 180°, ladite ouverture en V étant orientée, soit vers le bas pour concentrer les champs électromagnétiques, soit vers le haut pour disperser lesdits champs électromagnétiques et **en ce qu'**un support isolant primaire (8; 9), respectivement complémentaire (8'; 9'), d'un circuit oscillant primaire (3, 6 ou 10), respectivement complémentaire (3', 6' ou 10'), apte à neutraliser les rayonnements électromagnétiques telluriques dans les micro-ondes est, de préférence, situé dans ledit plan de référence (P).

18. Dispositif, selon l'une quelconque des revendications 1 à 17, **caractérisé en ce que** la capacité C de chaque circuit oscillant LC primaire (3, 6 ; 10), respectivement complémentaire (3', 6' ; 10'), apte à neutraliser les rayonnements électromagnétiques d'origine tellurique dans les micro-ondes, est constitué par la capacité parasite dudit circuit oscillant LC primaire (3, 6 ; 10), respectivement complémentaire (3', 6' ; 10').

19. Dispositif, selon l'une quelconque des revendications 2 à 17, **caractérisé en ce que** chaque circuit oscillant complémentaire (1', 2'; 3', 4'; 5', 6' ou 10') est relié, électriquement, au circuit oscillant primaire (1, 2; 3, 4; 5, 6 ou 10) auquel il est associé, par l'intermédiaire de l'une des extrémités de la self complémentaire (1'; 2'; 3' ou 10'), à l'une des extrémités, intérieure ou extérieure, de la self primaire (1 ; 2 ; 3 ou 10).

**20.** Dispositif, selon l'une quelconque des revendications 1 à 20, **caractérisé en ce que** chaque circuit oscillant primaire (1, 2; 3, 4; 5, 6 ou 10), respectivement complémentaire (1', 2'; 3', 4'; 5', 6' ou 10'), est coulé dans une résine isolante.

**21.** Dispositif, selon l'une quelconque des revendications 1 à 20, **caractérisé en ce que** les circuits oscillants primaires (1, 4, et 2, 5), et le cas échéant complémentaires (1', 4' et 2', 5'), aptes à neutraliser les rayonnements électromagnétiques dans les bandes de fréquences VHF et UHF comportent en outre des selfs extérieures, comprenant une ou deux spires chacune, arrangées respectivement autour des selfs primaires (1 ou 2), et le cas échéant complémentaires (1' ou 2'), desdits circuits oscillants et munies à leurs extrémités de brins rayonnants ou analogues.

## Patentansprüche

**1.** Passives elektronisches Gerät, das dafür vorgesehen ist, gegen kapillare Anstiege in Mauern und Fundamenten zu deren Trocknung zu wirken, wobei das Gerät als funktionelle Komponenten im Wesentlichen LC-Schwingkreise aufweist, wobei die Schwingkreise (1, 4; 2, 5; 3, 6 oder 10) voneinander unabhängig sind, in einer für elektromagnetische Felder durchlässigen Schutzbox angeordnet sind und sich jeweils aus einer Primärdrossel (1; 2; 3 oder 10) zusammensetzen, die elektrisch mit einem Primärkondensator (4, 5, 6) verbunden ist und/oder der parasitären Kapazität des in Betracht gezogenen Kreises zugeordnet ist, und wobei die Primärdrosseln (1; 2; 3 oder 10) mit im Wesentlichen flacher Struktur ungefähr in ein und derselben Referenzebene (P) angeordnet sind, wobei das Gerät **gekennzeichnet ist dadurch**, dass die Schwingkreise (1, 4; 2, 5; 3, 6 oder 10) auf isolierenden Trägern montiert sind, **dadurch**, dass es mindestens einen Primärschwingkreis (2, 5), der dazu geeignet ist, elektromagnetische Erdstrahlen im VHF-Frequenzband zu neutralisieren, mindestens einen Primärschwingkreis (1, 4), der dazu geeignet ist, elektromagnetische Erdstrahlen im UHF Frequenzband zu neutralisieren, und mindestens einen Primärschwingkreis (3, 6; 10) aufweist, der dazu geeignet ist, elektromagnetische Erdstrahlen im Mikrowellenbereich zu neutralisieren, **dadurch**, dass jedem Primärschwingkreis (1, 4; 2, 5; 3, 6 oder 10) jeweils mindestens ein Komplementärschwingkreis (1', 4'; 2', 5'; 3', 6' oder 10') zugeordnet ist, von welchen jeder eine Komplementärdrossel (1', 2', 3' oder 10') aufweist, die elektrisch mit einem Komplementärkondensator (4', 5', 6') verbunden ist und/oder der parasitären Kapazität des betrachteten Kreises zugeordnet ist, und **dadurch**, dass jede Komplementärdrossel (1'; 2'; 3' oder 10') im Wesentlichen parallel zu der jeweiligen Primärdrossel (1; 2; 3 oder 10) des entsprechenden Primärschwingkreises (1, 4; 2, 5; 3, 6 oder 10) angeordnet ist und entsprechend der angestrebten Strahlungscharakteristik und der zu neutralisierenden Frequenzen eine Struktur und eine Anordnung bezüglich der entsprechenden Primärdrossel (1; 2; 3 oder 10) aufweist.

**2.** Gerät nach Anspruch 1, **gekennzeichnet dadurch, dass** die Anzahl der Windungen jeder Primärdrossel (1, 2) bzw. jeder Komplementärdrossel (1', 2') eines Primärschwingkreises (1, 4; 2, 5) bzw. eines Komplementärschwingkreises (1', 4'; 2', 5'), der dazu geeignet ist, die elektromagnetischen Erdstrahlen im entsprechenden VHF- oder UHF-Frequenzband zu neutralisieren, zwischen 8 und 60 Windungen, vorzugsweise zwischen 10 und 48 Windungen, liegt und der Wert der Kapazität eines Primärkondensators (4, 5) bzw. eines Komplementärkondensators (4', 5'), der elektrisch mit der Primärdrossel (1, 2) bzw. mit der Komplementärdrossel (1', 2') verbunden ist, vorteilhaft zwischen 0,01 und 3$\mu$F, vorzugsweise zwischen 0,1 und 2$\mu$F, liegt und dadurch, dass die Anzahl der Windungen der Primärdrossel (3) bzw. der Komplementärdrossel (3') des Primärschwingkreises (3, 6) bzw. des Komplementärschwingkreises (3', 6'), der dazu geeignet ist, Strahlungen im Mikrowellenbereich zu neutralisieren, zwischen 1 und 5 Windungen, vorzugsweise zwischen 1 und 3 Windungen, liegt und der Wert der Kapazität des Primärkondensators (6) bzw. des Komplementärkondensators (6'), der elektrisch mit der Primärdrossel (3) bzw. der Komplementärdrossel (3') elektrisch verbunden ist oder dieser Drossel (3, 3') in Form einer parasitären Kapazität zugeordnet ist, zwischen 1 und 22pF liegt.

**3.** Gerät nach Anspruch 2, **gekennzeichnet dadurch, dass** der Durchmesser jeder Primärdrossel (1, 2, 3) bzw. jeder Komplementärdrossel (1', 2', 3') zwischen 50 und 260 mm liegt, und dadurch, dass die Schrittweite jeder Primärdrossel (1, 2, 3) bzw. jeder Komplementärdrossel (1', 2', 3') zwischen 0,2 und 6 mm liegt.

**4.** Gerät nach einem der Ansprüche 1 bis 3, **gekennzeichnet dadurch, dass** jeder Primärschwingkreis (1, 4; 2, 5; 3, 6 oder 10) bzw. jeder Komplementärschwingkreis (1', 4'; 2', 5'; 3', 6' oder 10') auf einem isolierenden, ebenen Primärträger (8) bzw. Komplementärträger (8') derart montiert ist, dass jede Primärdrossel (1; 2; 3 oder 10) bzw. jede Komplementärdrossel (1'; 2'; 3' oder 10) eine ebene Spiralstruktur aufweist.

**5.** Gerät nach Anspruch 4, **gekennzeichnet dadurch, dass** die ebene Spiralstruktur jeder Primärdrossel (1; 2; 3 oder 10) bzw. jeder Komplementärdrossel (1'; 2'; 3' oder 10') ausgehend von mindestens einem Kupferleiter oder mindestens einem folierten, versilberten oder vergoldeten Kupferband realisiert ist, der oder das unmittelbar auf dem entsprechenden isolierenden, ebenen Primärträger (8) bzw. Komplementärträger (8') angeordnet und spiralförmig gewickelt ist.

**6.** Gerät nach Anspruch 4 oder nach Anspruch 5, **gekennzeichnet dadurch, dass** jeder Primärkondensator (4, 5, 6) bzw. jeder Komplementärkondensator (4', 5', 6') elektrisch mit dem Zentrum und dem freien Ende der entsprechenden Primärdrossel (1, 2, 3) bzw. der Komplementärdrossel (1', 2', 3') des betreffenden Schwingkreises verbunden ist.

**7.** Gerät nach Anspruch 4 oder nach Anspruch 5, **gekennzeichnet dadurch, dass** die ebene Spiralstruktur der Primärdrossel (10) bzw. der Komplementärdrossel (10') mindestens eines Primärschwingkreises (10) bzw. Komplementärschwingkreises (10'), der dazu geeignet ist, die elektromagnetischen Erdstrahlungen im Mikrowellenbereich zu neutralisieren, mehrere Spiralarme (30) aufweist, die wechselweise ineinander verschachtelt sind und untereinander bei ihren innenliegenden Enden elektrisch verbunden sind, wodurch der zentraler Drehpunkt oder das Zentrum (30') der Spiralarme (3) gebildet wird, und dadurch, dass einerseits jeder Spiralarm (30) sich über 1 bis 3 Umläufe erstreckt und sich andererseits die Schrittweite der dadurch erhaltenen Spiralstruktur gleich bleibend ist oder sich ausgehend von dem zentralen Punkt (30') zunehmend vergrößert.

**8.** Gerät nach Anspruch 7, **gekennzeichnet dadurch, dass** die Primärdrossel (10) und die Komplementärdrossel (10') identisch sind und jede jeweils einen Primärschwingkreis oder einen Komplementärschwingkreis festlegt, dies in Verbindung mit einer entsprechenden parasitären Kapazität, die im Bereich des Kreises vorhanden ist.

**9.** Gerät nach Anspruch 6 oder Anspruch 8, **gekennzeichnet dadurch, dass** das Zentrum einer Primärdrossel (1, 2, 3, 10) bzw. einer Komplementärdrossel (1', 2', 3', 10') ferner mit einer metallischen Leiterstange, einem Ferritstab (7), einem Magnetstab oder einem Quarz von einigen Millimetern bis zu einigen Zentimetern Länge verbunden ist.

**10.** Gerät nach Anspruch 9, **gekennzeichnet dadurch, dass** die metallische Leiterstange, der Ferritstab (7), der Magnetstab oder der Quarz senkrecht zum entsprechenden isolierenden, ebenen Primärträger (8) bzw. Komplementärträger (8') angeordnet ist oder derart senkrecht im isolierenden, ebenen Primärträger (8) bzw. Komplementärträger (8') integriert ist, dass die Länge welche über den isolierenden, ebenen Primärträger (8) bzw. Komplementärträger (8') hinausragt, zwischen ungefähr 40 und 100% der Gesamtlänge liegt.

**11.** Gerät nach einem der Ansprüche 1 bis 3, **gekennzeichnet dadurch, dass** jede Primärdrossel (1, 2, 3) bzw. Komplementärdrossel (1', 2', 3') eine zylindrische Spiralstruktur aufweist und dadurch, dass die Windungen jeder Primärdrossel (1, 2, 3) bzw. Komplementärdrossel (1', 2', 3') auf einem isolierenden zylindrischen Primärträger (9) bzw. Komplementärträger (9') aufgewikkelt sind.

**12.** Gerät nach einem der Ansprüche 1 bis 3, **gekennzeichnet dadurch, dass** jede Primärdrossel (1, 2, 3) bzw. Komplementärdrossel (1', 2', 3') eine ebene zylindrische Spiralstruktur aufweist und dadurch, dass die Windungen jeder Primärdrossel (1, 2, 3) bzw. Komplementärdrossel (1', 2', 3') sich aus einem festen und selbsttragenden Draht zusammensetzen.

**13.** Gerät nach Anspruch 11 oder nach Anspruch 12, **gekennzeichnet dadurch, dass** jeder Primärkondensator (4, 5, 6) bzw. Komplementärkondensator (4', 5', 6') elektrisch mit unteren und oberen Enden der entsprechenden Primärdrossel (1, 2, 3) bzw. Komplementärdrossel (1', 2', 3') des betreffenden Schwingkreises verbunden ist.

**14.** Gerät nach einem der Ansprüche 11 bis 13, **gekennzeichnet dadurch, dass** ein Ferritstab, ein Magnetstab oder ein Quarz von einigen Millimetern bis zu einigen Zentimetern Länge im Wesentlichen in axialer Richtung im Inneren der ebenen zylindrischen Struktur jeder Primärdrossel (1, 2, 3) bzw. Komplementärdrossel (1', 2', 3') angeordnet ist.

**15.** Gerät nach einem der Ansprüche 11 bis 14, **gekennzeichnet dadurch, dass** eine metallische Leiterstange am oberen Ende jeder entsprechenden Primärdrossel (1, 2, 3) bzw. Komplementärdrossel (1', 2', 3') des betreffenden Schwingkreises befestigt ist.

**16.** Gerät nach einem der Ansprüche 1 bis 15, **gekennzeichnet dadurch, dass** die isolierenden Primärträger (8; 9) bzw. Komplementärträger (8'; 9') der Primärschwingkreise (1, 2, 4, 5) bzw. Komplementärschwingkreise (1', 2'; 4', 5'), die dazu geeignet sind, die elektromagnetischen Erdstrahlen in den VHF- und UHF-Frequenzbändern zu neutralisieren, unter einem Winkel bezüglich der Referenzebene (P) geneigt sind, der nahe oder im Wesentlichen gleich 0° ist, und dadurch, dass ein isolierender Primärträger (8; 9) bzw. Komplementärträger (8'; 9') eines Primärschwingkreises (3, 6 oder 10) bzw. Komplementärschwingkreises (3', 6' oder 10'), der dazu geeignet ist, elektromagnetische Erdstrahlen im Mikrowellenbereich zu neutralisieren, vorzugsweise in der Referenzebene (P) angeordnet ist.

17. Gerät nach einem der Ansprüche 1 bis 15, **gekennzeichnet dadurch, dass** die isolierenden Primärträger (8; 9) bzw. Komplementärträger (8'; 9') der Primärschwingkreise (1, 2; 4, 5) bzw. Komplementärschwingkreise (1', 2'; 4', 5'), die dazu geeignet sind, elektromagnetische Erdstrahlen in den VHF- und UHF-Frequenzbändern zu neutralisieren, unter einem Winkel (α) von ungefähr 1° bis 30°, vorzugsweise von 1° bis 20°, bezüglich der Referenzebene (P) geneigt sind, dies in einer Art und Weise, um eine V-Konfiguration zwischen den isolierenden Primärträgern (8; 9) bzw. Komplementärträgern (8'; 9') mit einem ungefähren Winkel(ß) von 120° bis 180° zu formen, wobei die V-Öffnung entweder nach unten, um die elektromagnetischen Felder zu konzentrieren, oder nach oben orientiert ist, um die elektromagnetischen Felder zu dispersieren, und dadurch, dass ein isolierender Primärträger (8; 9) bzw. Komplementärträger (8'; 9') eines Primärschwingkreises (3, 6 oder 10) bzw. Komplementärschwingkreises (3', 6' oder 10'), die dazu geeignet sind, elektromagnetische Erdstrahlen im Mikrowellenbereich zu neutralisieren, vorzugsweise in der Referenzebene (P) angeordnet ist.

18. Gerät nach einem der Ansprüche 1 bis 17, **gekennzeichnet dadurch, dass** die Kapazität C jedes Primärschwingkreises LC (3, 6; 10) bzw. Komplementärschwingkreises (3', 6'; 10'), der dazu geeignet ist, elektromagnetische Mikrowellen-Erdstrahlen zu neutralisieren, durch eine parasitäre Kapazität des Primärschwingkreises LC (3, 6; 10) bzw. Komplementärschwingkreises (3', 6'; 10') gebildet wird.

19. Gerät nach einem der Ansprüche 2 bis 17, **gekennzeichnet dadurch, dass** jeder Komplementärschwingkreis (1', 2'; 3', 4'; 5', 6' oder 10') elektrisch mit dem Primärschwingkreis (1, 2; 3, 4; 5, 6 oder 10), welchem er zugeordnet ist, über eines der Enden der Komplementärdrossel (1'; 2'; 3' oder 10') und eines der inneren oder äußeren Enden der Primärdrossel (1; 2; 3 oder 10) verbunden ist.

20. Gerät nach einem der Ansprüche 1 bis 20, **gekennzeichnet dadurch, dass** jeder Primärschwingkreis (1, 2; 3, 4; 5, 6 oder 10) bzw. Komplementärschwingkreis (1', 2'; 3'; 4'; 5', 6' oder 10') in einen Isolierlack gegossen ist.

21. Gerät nach einem der Ansprüche 1 bis 20, **gekennzeichnet dadurch, dass** die Primärschwingkreise (1, 4 und 2, 5) und gegebenenfalls die Komplementärschwingkreise (1', 4' und 2', 5'), die dazu geeignet sind, die elektromagnetischen Strahlungen in den VHF- und UHF-Frequenzbändern zu neutralisieren, ferner externe Drosseln aufweisen, welche jede ein oder mehrere Windungen aufweisen, die jeweils um die Primärdrosseln (1 oder 2) und gegebenenfalls die Komplementärdrosseln (1' oder 2') der Schwingkreise herum angeordnet sind, wobei die besagten Schwingkreise an ihren Enden mit Abstrahlstangen oder ähnlichem versehen sind.

## Claims

1. Passive electronic device intended to act against capillary rise in walls and foundations, for the purpose of drying the latter, said device comprising mainly LC oscillating circuits, as functional components, said oscillating circuits (1, 4; 2, 5; 3, 6 or 10) being independent from one another, housed in a protective housing permeable to electromagnetic fields and each consisting of a primary self-inducting coil (1; 2; 3 or 10) connected electrically to a primary capacitor (4, 5, 6) and/or connected to the parasitic capacitance of the circuit concerned and in that said primary self-inducting coils (1; 2; 3 or 10), with a substantially flat structure, are located approximately in the same reference plane (P),
the device being **characterised in that**
said oscillating circuits (1, 4; 2, 5; 3, 6 or 10) are mounted on insulating bases,
**in that** it comprises at least one primary oscillating circuit (2, 5) able to neutralise the electromagnetic radiation of telluric origin in the VHF frequency band, at least one primary oscillating circuit (1, 4) able to neutralise the electromagnetic radiation of telluric origin in the UHF frequency band and at least one primary oscillating circuit (3, 6; 10) able to neutralise the electromagnetic radiation of telluric origin in the microwave range,
**in that** to each primary oscillating circuit (1, 4; 2, 5; 3, 6 or 10) at least one complementary oscillating circuit (1', 4'; 2', 5'; 3', 6' or 10') is connected respectively, each comprising a complementary self-inducting coil (1', 2', 3' or 10') connected electrically to a complementary capacitor (4', 5', 6') and/or connected to the parasitic capacitance of the circuit concerned and
**in that** each complementary self-inducting coil (1'; 2'; 3' or 10') is arranged substantially parallel to the primary self-inducting coil (1; 2; 3 or 10) connected with the corresponding primary oscillating circuit (1, 4; 2, 5; 3, 6 or 10) and has a structure and a position relative to the corresponding primary self-inducting coil (1; 2; 3 or 10) which are dependent on the desired radiation pattern and frequencies to be neutralised.

2. Device according to claim 1, **characterised in that** the number of turns of each primary self-inducting coil (1, 2), complementary coil (1', 2') respectively, of a primary oscillating circuit (1, 4; 2, 5), complementary circuit (1', 4'; 2', 5') respectively, able to neutralise the electromagnetic radiation of telluric origin in the corresponding VHF or UHF frequency band is between 8 and 60 turns, preferably between 10 and 48 turns, and the value of the capacity of a primary capacitor (4, 5), complementary capacitor (4', 5') respectively connected electrically to the said primary self-inducting coil (1, 2), complementary coil (1', 2') respectively, is advantageously between 0.01 and 3 $\mu$F, preferably between 0.1 and 2 $\mu$F, and **in that** the number of turns of the primary self-inducting coil (3), complementary coil (3') respectively, of the primary oscillating circuit (3, 6), complementary circuit (3', 6') respectively, able to neutralise radiation in the microwave range, is between 1 and 5 turns, preferably between 1 and 3 turns, and the value of the capacity of the primary capacitor (6), complementary capacitor (6') respectively, connected electrically to the said primary self-inducting coil (3), complementary coil (3') respectively, or connected to said self-inducting coil (3, 3') in the form of a parasitic capacitor is between 1 and 22 pF.

3. Device according to claim 2, **characterised in that** the diameter of each primary self-inducting coil (1, 2, 3), complementary coil (1', 2', 3') respectively, is between 50 and 260 mm and **in that** the pitch of each primary self-inducting coil (1, 2, 3), complementary coil (1', 2', 3') respectively, is between 0.2 and 6 mm.

4. Device according to any one of claims 1 to 3, **characterised in that** each primary oscillating circuit (1, 4; 2, 5; 3, 6 or 10), complementary circuit (1', 4'; 2', 5'; 3', 6' or 10') respectively, is mounted on a primary planar insulating base (8), complementary base (8') respectively, such that each primary self-inducting coil (1; 2; 3 or 10), complementary coil (1'; 2'; 3' or 10') respectively, has a planar spiral structure.

5. Device according to claim 4, **characterised in that** the planar spiral structure of each primary self-inducting coil (1; 2; 3 or 10), complementary coil (1'; 2'; 3' or 10') respectively, is in the form of at least one copper conductor or at least one tin-plated, silver-plated or gold-plated copper band, arranged like the primary planar insulating base (8), complementary (8') base respectively, and wound into a spiral.

6. Device according to claim 4 or claim 5, **characterised in that** each primary capacitor (4, 5, 6), complementary capacitor (4', 5', 6') respectively, is connected electrically to the centre and free end of the corresponding primary self-inducting coil (1, 2, 3), complementary coil (1', 2', 3') respectively, of the oscillating circuit concerned.

7. Device according to claim 4 or claim 5, **characterised in that** the planar spiral structure of the primary self-inducting coil (10), complementary coil (10') respectively, of the at least one primary oscillating circuit (10), complementary circuit (10') respectively, able to neutralise electromagnetic radiation of telluric origin in the microwave range comprises a plurality of spiralled arms (30) imbricated with one another in an alternating manner and connected electrically to one another at their inner end forming the central point of revolution or centre (30') of said spiral arms (3), and **in that**, on the one hand, each spiral arm (30) extends over 1 to 3 turns and, on the other hand, the pitch of the spiral structure obtained in this way is constant or increases progressively from the said central point (30').

8. Device according to claim 7, **characterised in that** the primary (10) and complementary (10') self-inducting coils are identical and each form respectively a primary oscillating circuit or a complementary oscillating circuit, in association with a corresponding parasitic capacity of said circuit.

9. Device according to claim 6 or claim 8, **characterised in that** the centre of a primary (1, 2, 3, 10) or complementary (1', 2', 3', 10') self-inducting coil is also connected to a metallic conducting core, a ferrite bar (7), a bar magnet or quartz, of from several millimetres to several centimetres in length.

10. Device according to claim 9, **characterised in that** the metallic conducting core, the ferrite bar (7), the bar magnet or quartz is positioned to be perpendicular to the corresponding primary planar insulating base (8), complementary base (8') respectively, or is integrated perpendicularly into the said primary planar insulating base (8), complementary base (8') respectively, such that the length emerging above the primary planar insulating base (8), complementary base (8') respectively, is between about 40 and 100% of the total length.

11. Device according to any one of claims 1 to 3, **characterised in that** each primary self-inducting coil (1, 2, 3), complementary coil (1', 2', 3') respectively, has a cylindrical spiral structure and **in that** the turns of each primary self-inducting coil (1, 2, 3), complementary coil (1', 2', 3') respectively, are wound onto a primary cylindrical insulating base (9), complementary base (9') respectively.

12. Device according to any one of claims 1 to 3, **char-**

**acterised in that** each primary self-inducting coil (1, 2, 3), complementary coil (1', 2', 3') respectively, has a flat cylindrical spiral structure and **in that** the turns of each primary self-inducting coil (1, 2, 3), complementary coil (1', 2', 3') respectively, are formed by a rigid and self-supporting wire.

13. Device according to claim 11 or claim 12, **characterised in that** each primary capacitor (4, 5, 6), complementary capacitor (4', 5', 6') respectively, is connected electrically to the corresponding lower end and the upper end of the primary self-inducting coil (1, 2, 3), complementary coil (1', 2', 3') respectively, of the oscillating circuit concerned.

14. Device according to any one of claims 11 to 13, **characterised in that** a ferrite bar, a bar magnet or quartz, of from several millimetres to several centimetres in length, is positioned, substantially axially, relative to the inside of the flat cylindrical structure of each primary self-inducting coil (1, 2, 3), complementary coil (1', 2', 3') respectively.

15. Device according to any one of claims 11 to 14, **characterised in that** a metal conducting core is fixed to the upper end of each corresponding primary self-inducting coil (1, 2, 3), complementary coil (1', 2', 3') respectively, of the oscillating circuit concerned.

16. Device according to any one of claims 1 to 15, **characterised in that** the primary insulating bases (8; 9), complementary bases (8', 9') respectively, of the primary oscillating circuits (1, 2; 4, 5), complementary circuits (1', 2'; 4', 5') respectively, able to neutralise the telluric electromagnetic radiation in the VHF and UHF frequency bands, are inclined at an angle close to or substantially equal to 0° relative to the reference plane (P) and **in that** a primary insulating base (8; 9), complementary base (8'; 9') respectively, of a primary oscillating circuit (3, 6, or 10), complementary circuit (3', 6' or 10') respectively, able to neutralise telluric electromagnetic radiation in the microwave range, is preferably located in the said reference plane (P).

17. Device according to any one of claims 1 to 15, **characterised in that** the primary insulating bases (8; 9), complementary bases (8'; 9') respectively, of the primary oscillating circuits (1, 2; 4, 5), complementary circuits (1', 2'; 4', 5') respectively, able to neutralise telluric electromagnetic radiation in the VHF and UHF frequency band, are inclined at an angle (α) of approximately from 1° to 30°, preferably from 1° to 20°, relative to the reference plane (P), in such a way as to form a V configuration between said primary insulating bases (8; 9), complementary bases (8'; 9) respectively, of an angle (β) of approximately from 120° to 180°, said V opening being oriented either towards the bottom to concentrate the electromagnetic fields, or towards the top to disperse said electromagnetic fields and **in that** a primary insulating base (8; 9), complementary base (8'; 9') respectively, of a primary oscillating circuit (3, 6, or 10), complementary circuit (3', 6' or 10') respectively, able to neutralise telluric electromagnetic radiation in the microwave range, is preferably located in the said reference plane (P).

18. Device according to any one of claims 1 to 17, **characterised in that** the capacity C of each primary LC oscillating circuit (3, 6; 10), complementary circuit (3', 6'; 10') respectively, able to neutralise electromagnetic radiation of telluric origin in the microwave range, is formed by the parasitic capacity of the said primary LC oscillating circuit (3, 6; 10), complementary circuit (3', 6'; 10') respectively.

19. Device according to any one of claims 2 to 17, **characterised in that** each complementary oscillating circuit (1', 2'; 3'; 4'; 5', 6' or 10') is connected electrically to the primary oscillating circuit (1, 2; 3, 4; 5, 6 or 10) to which it is connected, by means of one of the ends of the complementary self-inducting coil (1'; 2'; 3' or 10'), to one of the inner or outer ends of the primary self-inducting coil (1; 2; 3 or 10).

20. Device according to any one of claims 1 to 20, **characterised in that** each primary oscillating circuit (1, 2; 3, 4; 5, 6 or 10), complementary circuit (1', 2'; 3', 4'; 5', 6' or 10') respectively, is cast in an insulating resin.

21. Device according to any one of claims 1 to 20, **characterised in that** the primary oscillating circuits (1, 4 and 2, 5), and possibly complementary circuits (1', 4' and 2', 5'), able to neutralise electromagnetic radiation in the VHF and UHF frequency bands also comprise outer self-inducting coils, each comprising one or two turns, arranged respectively around the primary self-inducting coils (1 or 2), and possible complementary coils (1' or 2'), of said oscillating circuits and provided at their ends with radiating strands or the like.

Fig.1

Fig.2a

Fig.2b

Fig.2c

15

Fig.3a

Fig.3b

Fig.3c

Fig.4a          Fig.4b

EP 2 204 512 B1

Fig.5

Fig.6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0152510 A

- EP 0354150 A